# EUROPEAN PATENT APPLICATION

(11) **EP 4 527 324 A1**
(43) Date of publication of application: **26.03.2025**
(21) Application number: 23199552.3
(22) Date of filing: 25.09.2023
(51) Int. Cl.: A61B 17/34, A61B 34/30, A61B 34/10, A61B 90/13

(54) **PUNCTURE GUIDE UNIT AND COMPUTER PROGRAM**

(71) Applicant: Atlas Medical Technologies GmbH, 44866 Bochum (DE)
(72) Inventor: KRÜGER, Timo, 44866 Bochum (DE)
(74) Representative: Eisenführ Speiser

(57) **Abstract**

The present invention relates to a puncture guiding unit (306) for a puncture guiding system for assisting a user in carrying out a puncture with of a patient a medical instrument on the patient's skin surface at a puncture position (202) and along a puncture orientation (204) is proposed. The puncture guiding unit (306) comprises an input unit (320), a position determination unit (322) and an output unit (324). The input unit (320) is configured for receiving planning data (214) indicative of the puncture position (202) and/or the orientation (204) in a coordinate system of an imaging system (100). The position determination unit (322) is configured for determining for an optical target device (302) having a field of view with a viewing axis a target position and/or orientation for the optical target device (302) in the coordinate system of the imaging system at which the puncture position (202) and/or the puncture orientation (204) relative to the skin surface of the patient lie on or are at least close to the viewing axis using the planning data. The output unit (324) that is configured for providing guidance and/or selection data (326) indicative of the determined target position and/or orientation of the optical target device (302) relative to the patient.

## Description

The present invention relates to a puncture guiding unit for a puncture guiding system for assisting a user in carrying out a puncture of a patient with a medical instrument on the patient's skin surface at a puncture position and along a puncture orientation. Moreover, the present invention relates to a puncture guiding system for assisting a user in carrying out a puncture with a medical instrument at a puncture position and along a puncture orientation of a patient's skin surface. Furthermore, the present invention relates to a puncture assistance system for assisting a user in carrying out a puncture with a medical instrument at a puncture position and along a puncture orientation of a patient's skin surface. The present invention also relates to a method for assisting a user in carrying out a puncture with a medical instrument at a puncture position and along a puncture orientation of a patient's skin surface. Furthermore, the present invention relates to a computer program for assisting a user in carrying out a puncture with a medical instrument at a puncture position and along a puncture orientation of a patient's skin surface and to a non-transitory computer-readable data medium storing the computer program. The present invention also relates to a use of the puncture guiding unit, the puncture guiding system and the puncture assistance system for assisting a user in carrying out a puncture of a patient with a medical instrument.

### BACKGROUND OF THE INVENTION

A puncture typically includes piercing a patient's skin, inserting a medical instrument into a body of a patient and guiding the instrument to a target location inside the patient's body. At the target location inside the patient, for example, diagnostic material can be obtained, diagnostically or therapeutically relevant substances can be injected, or pathological fluid accumulations can be removed.

In order to reduce a risk of harming sensitive body tissue, e.g., a nerve or an organ, a puncture is regularly carried out under visual control. A puncture under visual control typically comprises that the position and orientation of the medical instrument inside the patient's body is recorded using an imaging system, such as computed tomography scanner, a magnetic resonance imaging scanner, or a sonography scanner.

A puncture under visual control can be complemented with a needle positioning system that can be used to display, and especially mark, the insertion point and insertion angle of the medical instrument on the patient's skin surface. For example, DE 10 2005 024 157 A1 discloses a needle positioning device for positioning a medical instrument within a puncture area. The needle positioning device can be used to mark with directed electromagnetic radiation an access point and a relative orientation of a medical instrument.

US 2011/0257508 discloses a device for supporting, scanning, tomographically displaying a patient and carrying out an intervention. In an embodiment, the device includes a computer system to: calculate the spatial position of at least one intervention channel in spatial relation to the patient couch and/or to the patient on the basis of previously obtained positional information on the tomographic display of the patient; output the respectively current optical recordings of the patient on the patient couch online; and superpose the current optical recordings of the patient with a spatially adapted display of the intervention channel and/or intervention target point and/or an intervention instrument directed at the intervention channel.

Furthermore, US 2022/0409290 A1 discloses a method and a system for displaying an insertion point for a medical instrument into an object. For example, in the method, a marker is provided on a surface of the object that can be detected both tomographically and optically. Fluoroscopic or/and tomographic image data are generated that can be used to reconstruct a fluoroscopic or/and tomographic image of the marker together with the object. An insertion point for the medical instrument is determined on the surface of the object relative to the marker in the coordinate system of the fluoroscopic or/and tomographic image data. Visual image data are generated that can be used to reconstruct a visual image of the at least one marker together with the object. The coordinate of the insertion point is transformed in the coordinate system of the fluoroscopic or/and tomographic image data into the coordinate system of the visual image data using the relative position of the insertion point to the at least one marker. The insertion point for the medical instrument is then displayed in real-time in a view of the object. Thereby, a user can reliably and precisely puncture the object based on the real-time display of the insertion point in the view of the object.

Yet, there is an ongoing demand of further simplifying and improving the way a puncture of a patient is carried out. In particular, the complexity of carrying out a puncture and, at the same time, a risk of an incorrect puncture and harm resulting from it shall be reduced.

### SUMMARY OF THE INVENTION

The present invention is based on the objective of providing an improved puncture guiding unit, an improved puncture guiding system and/or an improved puncture assistance system for assisting a user in carrying out a puncture with a medical instrument. Furthermore, the present invention is based on the objective of providing an improved method for assisting a user in carrying out a puncture with a medical instrument. The present invention is also based on the objective of providing an improved computer program for assisting a user in carrying out a puncture with a medical instrument.

According to the invention, a puncture guiding unit for a puncture guiding system for assisting a user in carrying out a puncture with of a patient a medical instrument on the patient's skin surface at a puncture position and along a puncture orientation is proposed. The puncture guiding unit comprises an input unit, a position determination unit and an output unit. The input unit is configured for receiving planning data indicative of the puncture position and/or the orientation in a coordinate system of an imaging system. The position determination unit is configured for determining a target position and orientation for an optical target device having a field of view with a viewing axis using the planning data. The target position and orientation is a position and/or orientation for the optical target device in the coordinate system of the imaging system. The target position and orientation is a position at which the viewing axis of the optical target device at least approximately coincides with a puncture trajectory determined according to the planning data. In other words, when the optical target device is located at the target position and orientation, the puncture position and/or the puncture orientation relative to the skin surface of the patient lie on or are at least close to the viewing axis. The output unit is configured for providing guidance and/or selection data indicative of the determined target position and/or orientation of the optical target device relative to the patient.

Once the optical target device is positioned at its target position relative to a patient, a user can place an instrument or a needle between the optical target device and a patient's skin. Once the longitudinal axis of the needle or instrument coincides with the viewing axis of the optical target device - i.e. once the user sees the needle or the instrument exactly from behind - the needle or instrument is positioned correctly for a puncture as planned.

The present invention includes the recognition that in case several tomographic images have to be generated during a puncture in order to check the progress of introducing a medical instrument into a patient, both, the surgeon as well as the patient are exposed to a comparatively large amount of X-ray radiation. Moreover, the puncture needs to be interrupted each time a tomographic image is generated such that the overall time for carrying out the puncture is comparatively long. It is therefore beneficial if the puncture can be carried out outside the imaging system and with a comparatively little number of tomographic images to be generated. Ideally, only one tomographic image should be generated at the beginning for planning the puncture. Thereby, the overall X-ray exposure of the surgeon and the patient can be significantly reduced.

However, if a puncture is to be carried out outside the imaging system and thus without visual control by means of the imaging system, it needs to be ensured that the surgeon can find the planned puncture position and can introduce the medical instrument at the planned puncture position along the planned puncture orientation. It is thus beneficial to provide assistance to a user, e.g., the surgeon, to carry out the puncture outside the imaging system and with only little or no additional generation of tomographic images.

With the puncture guiding unit according to the invention it is possible to determine based on planning data a target position and/or orientation for the optical target device in the coordinate system of the imaging system at which the puncture position and/orthe puncture orientation relative to the skin surface of the patient lie on or are at least close to the viewing axis using the planning data. The guidance and/or selection data indicative thereof can thus be used for positioning the optical target device at the determined target position and/or orientation. The puncture guiding unit can thus be used to assist a user in finding puncture position and/or the puncture orientation relative to the skin surface using the optical target device guided by or selected based on the guidance and/or selection data. Since the optical target device can be arranged at the determined target position and/or orientation at which the puncture position and/or the puncture orientation relative to the skin surface of the patient lie on or are at least close to the viewing axis using the planning data, a user can easily find puncture position and/or the puncture orientation relative to the viewing axis of the optical target device. That is, a user can align a medical instrument with the viewing axis of the optical target device and thereby finds the intended puncture position and/or the puncture orientation relative to the skin surface of the patient. Thereby the puncture guiding unit can assist a user in carrying out a puncture outside the imaging system and without the need of verifying the puncture in additionally generated tomographic images.

Aligning the medical instrument with the viewing axis is possible in that the medical instrument is moved by user into the field of view of the optical target device such that only a proximal end of the medical device is visible for the user. To this end, the optical target device is arranged at a distance to the patient that allows arranging the medical instrument in the field of view of the optical target device between the patient and the optical target device. If only a proximal end of the medical device is visible for the user, the medical instrument is aligned with the viewing axis. Since the viewing axis points towards the puncture positon on the skin surface of the patient and along puncture orientation, the aligned medical instrument can be moved along the viewing axis and thereby towards the puncture position in a comparatively simple and reliable manner to carry out the puncture.

In contrast to indicating the puncture position and puncture orientation with a laser, the alignment of a medical device in a field of view of an optical target device with the viewing axis has the advantage that the indicators for the puncture position and puncture orientation are not interrupted or modified by the medical instrument or body parts of the user. For example, a laser beam may be reflected or may be interrupted while trying to arranged the medical instrument relative to the laser beam. Thereby, a user may become uncertain about the correct positioning of the medical instrument. Moreover, once the medical instrument is arranged within the laser beam, the orientation of the medical instrument may need to be estimated by a user, in case the laser beam is blocked by the medical instrument.

Preferably, the guidance and/or selection data are suitable for controlling one or more actuators and/or brakes that are arranged and configured for guiding the optical target device to the determined target position and/or orientation of the optical target device relative to the patient. For example, the actuators and/or brakes may be part of an optical target device holder that holds the optical target device. Preferably, the optical target device holder is configured for enabling a positioning of the optical target device relative to the patient. The guidance and/or selection data may thus be used for controlling an optical target device holder such as a robot arm for positioning the optical target device in space relative to the patient such that the puncture position and/or the puncture orientation relative to the skin surface of the patient lie on or are at least close to the viewing axis.

Alternatively or in addition, the guidance and/or selection data may be suitable for indicating a direction along which the optical target device has to be moved in orderto bring the optical target device to the determined target position and/or orientation relative to the patient. For example, by indicating a direction along which the optical target device has to be moved to bring the optical target device to the determined target position and/or orientation relative to the patient, a user can be put into a position where the user can manually guide the optical target device to the determined target position and/or orientation based on the indicated direction. For example, based on the guidance and/or selection data, a visual signal, a haptic signal or an audio signal may be generated that is suitable for indicating a direction along which the optical target device has to be moved in order to bring the optical target device to the determined target position and/or orientation relative to the patient. It is also possible that actuators and/or brakes of an optical target device holder are controlled based on the guidance and/or selection data in a way that a further movement of the optical target device away from the determined target position and/or orientation is impeded by the actuators and/or brakes whereas a further movement of the optical target device towards the determined target position and/or orientation is enabled by the actuators and/or brakes.

Optionally, the guidance and/or selection data may be suitable for indicating that optical target device for which the determined target position and/or orientation of the optical target device relative to the patient is the closest to the viewing axis. For example, there may be a plurality of optical target devices arranged at stationary positions and based on the guidance and/or selection data it may be possible to select that optical target device out of the plurality optical target devices for which the puncture position and/or the puncture orientation relative to the skin surface of the patient lie the closest to the viewing axis of that optical target device.

According to the present invention, also a puncture guiding system for assisting a user in carrying out a puncture with a medical instrument at a puncture position and along a puncture orientation of the patient's skin surface is proposed. The puncture guiding system comprises an optical target device, an optical target device holder and the puncture guiding unit according to the present invention as described herein. The optical target device has a field of view associated therewith and a viewing axis, and may be, e.g., a camera or the like. The optical target device holder is configured for holding the optical target device relative to the patient, and may be, e.g., a robot arm or the like.

The puncture guiding system can be an integral part of an imaging system and may be fabricated together with the imaging system. It is also possible that the puncture guiding system is separate to an imaging system and can be arranged freely relative to the imaging system. In this case, when operating the puncture guiding system it may be necessary to register the puncture guiding system in the coordinate system of the imaging system. After registration of the puncture guiding system, a target position and/or orientation of the optical target device in the coordinate system of the imaging system at which the puncture position and/or the puncture orientation relative to the skin surface of the patient lie on or are at least close to a viewing axis of the optical target device using the planning data can be determined. Having determined the respective target position and/or orientation of the optical target device in the coordinate system of the imaging system, the optical target device holder can be used for positioning the optical target device, e.g., controlled based on guidance and/or selection data provided by the puncture guiding unit to assist a user in carrying out a puncture of the patient at the planned puncture positon.

A particular advantage of the puncture guiding system that it can be retrofitted to an imaging system such as a CT scanner or a MRI scanner. That is, an existing imaging system can be equipped with the puncture guiding system, and the optical target device holder may hold the optical target device relative to the imaging system. The puncture guiding system attached to the imaging system may need to be registered once with the imaging system. Possibly, since there may be some play or deviation, such registration of the attached puncture guiding system may need to be repeated in longer periods of time. In principle, however, it can be assumed for normal operation that the puncture guiding system is registered once and then stays registered with the imaging system. After registration, the position of the puncture guiding system is known in the coordinate system of the imaging system.

For positioning the optical target device such that the puncture position and/or the puncture orientation relative to the skin surface of the patient lie on or are at least close to the viewing axis, patient registration may be required. Generally, a patient is registered in the coordinate system of the imaging system once a tomographic image of the patient is generated. A patient can then be moved outside the imaging system, e.g., on a table such as a CT table of a CT scanner and its position can still be determined in the coordinate system of the imaging system. That is, the patient stays registered also when moving the patient outside, e.g., a gantry, using a table. However, in case a patient is expected to move after having generated the tomographic image, a further registration should be carried out to know the position and orientation of the patient at the time of the puncture.

For example, if the optical target device such as a camera is registered with the imaging system, the patient can be registered via the camera held by the optical target device holder. Alternatively, one or more separate cameras can be provided, e.g., attached to the imaging system, which are registered with the imaging system and which can perform the optical registration of the patient after the patient has been moved out of the imaging system.

Preferably, the optical target device is a portable electronic device and comprises a camera or the like. The camera may be configured for providing video data representing a video of that part of the world that can be seen through the camera. Preferably, the optical target device holder is configured as a robot arm with one or more joints. The robot arm may have several elements that are connected by the joints such that the several elements of the robot arm can be moved relative to one another, e.g., angled and straightened. The robot arm holding the optical target device can be used for positioning the optical target device relative to the imaging system and a patient arranged in the imaging system. In particular, the robot arm can be controlled based on guidance and/or selection data to position the optical target device relative to the patient such that the viewing axis runs towards the puncture position and along the puncture orientation relative to the skin surface of the patient .

Arranging the optical target device held by the robot arm can be carried out manually or automatically. For example, the robot arm comprises one or more brakes that are arranged and configured to be controlled by the puncture guiding unit, e.g., based on guidance and/or selection data, to impede further movement of the joints. When moving the optical target device attached to the robot arm manually, the brakes may be actuated in case a user moves the optical target device away from the determined target position and/or orientation of the optical target device at which the puncture position and/or the puncture orientation relative to the skin surface of the patient lie on or are at least close to the viewing axis. A user then receives haptic feedback that the optical target device is moved away from the intended position of the optical target device. Thereby, a user can be guided to correctly position the optical target device relative to the patient.

Alternatively or in addition, the robot arm may comprise one or more actuators, e.g., motors such as stepper motors, that are arranged and configured to be controlled by the puncture guiding unit, e.g., based on guidance and/or selection data, for actuating the joints to enable a further movement of the joints. A robot arm with actuators can be used to position the optical target device held by the robot arm fully automatically such that the viewing axis runs towards the puncture position and along the puncture orientation relative to the skin surface of the patient . Also a semi-automatic mode can be realized by combining, e.g., brakes configured for actuating the joints to impede a further movement of the joints and actuators configured for actuating the joints to enable a further movement of the joints.

It is also possible that the optical target device holder is configured as an arch element that is configured to movably hold the optical target device such that the optical target device can be moved along the arch element. Such an arch element can be arranged and configured such that a patient can be moved out of the imaging system on a table such that the patient is partly surrounded by the arch element. The optical target device can then be moved along the arch element such that the viewing axis points towards the puncture position and along the puncture orientation relative to the skin surface of the patient. Such an arch element may also have brakes and/or actuators that impede and enable a further movement of the optical target device along the arch element in order to guide a user to manually position the optical target device at the arch element. An arch element may also be equipped with brakes and/or actuators that may be controlled to automatically position the optical target device along the arch element.

Optionally, the optical target device holder may be configured as an arch element that is configured to hold one or more optical target devices at a stationary position relative to the arch element. For example, along the arch element, several optical target devices may be arranged and based on the guidance and/or selection data, that optical target device may be selected for which the viewing axis at least approximately points towards the puncture position and along the puncture orientation relative to the skin surface of the patient.

According to the present invention, also a puncture assistance system for assisting a user in carrying out a puncture with a medical instrument at a puncture position and along a puncture orientation on the patient's skin surface of a patient is proposed. The puncture assistance system comprises an imaging system, a planning unit and the puncture guiding system according to the present invention as described herein. The imaging system is configured for generating a tomographic image of a patient. The planning unit is configured for generating planning data indicative of the puncture position and/or the orientation in a coordinate system of the imaging system based on the tomographic image. For example, the imaging system may be a CT scanner and the planning unit may be connected to the CT scanner to receive a tomographic image. With the planning unit, a user can then plan the puncture to be carried out at a patient by selecting a puncture position and a puncture orientation. Optionally, a puncture depth may be selected. The result of the planning, i.e., the planned puncture position and the planned puncture orientation can then be provided by the planning unit in the coordinate system of the imaging system in form of planning data.

Preferably, the imaging system comprises a table that allows moving a patient outside the imaging system, e.g., outside a gantry or the like. Since the patient is registered once a tomographic image has been generated, the position of the patient in the coordinate system of the imaging system is known even after having moved the patient outside the imaging system. Moving a patient outside the imaging system partly refers to moving the patient outside the imaging modality that is used for generating the tomographic image such as a gantry. Since the movement of the table is known in the coordinate system of the imaging system, a further registration of the patient, in principle, is not required when the patient is moved outside the imaging system.

Moving the patient outside the imaging system may be desired because it allows to more freely position the medical instrument at the puncture position. Also some planned puncture positions may even only be accessible for a user if the patient is moved outside the imaging system. A further advantage of moving the patient outside the imaging system is that additional X-ray exposure can be avoided.

Yet, the patient's own movements relative to the table may require a renewed patient registration, preferably, an optical patient registration. It is thus preferred that the puncture assistance system is configured for performing a patient registration of the patient when the patient is moved out of the imaging system, e.g., out of a gantry or the like, on a table of the imaging system. To this end, the puncture assistance system may comprise an additional camera that is fixed to the imaging system such that the camera position is known in the coordinate system of the imaging system. Alternatively or in addition, the optical target device when comprising a camera may be used for registering the patient. This is possible as long as the position of the optical target device is known in the coordinate system of the imaging system. Preferably, the puncture guiding system is attached to the imaging system. If the puncture guiding system is attached to the imaging system, the position and orientation of the puncture guiding system is generally known in the coordinate system of the imaging system, e.g., after an initial registration.

According to the present invention, also a method for assisting a user in carrying out a puncture with a medical instrument at a puncture position and along a puncture orientation on the patient's skin surface of a patient is proposed. The method comprises the steps of:
- determining the puncture position and/or the puncture orientation in a coordinate system of an imaging system,
- determining a position and/orientation of an optical target device at which the viewing axis runs towards the puncture position and along the puncture orientation relative to the skin sur-face of the patient I, and
- guiding the optical target device to the determined puncture position and/or the puncture orientation such that the viewing axis runs towards the puncture position and along the puncture orientation relative to the skin surface of the patient, or
- selecting that optical target device for which the viewing axis runs towards the determined puncture position and along the puncture orientation relative to the skin surface of the patient.

The method can be carried out *inter alia* using the puncture assistance system described herein.

According to the present invention, also a computer program for assisting a user in carrying out a puncture with a medical instrument at a puncture position and along a puncture orientation of the patient's skin surface is proposed. The computer program is configured for executing the steps of:
- receiving planning data indicative of the puncture position and/or the orientation in the coordinate system of the imaging system,
- determining a target position and/or orientation of the optical target device in the coordinate system of the imaging system at which the viewing axis runs towards the puncture position and along the puncture orientation relative to the skin surface of the patient using the planning data, and
- providing guidance and/or selection data indicative of the determined target position and/or orientation of the optical target device relative to the patient, when run on a computer.

According to the present invention, also a non-transitory computer-readable data medium storing the computer program described herein is proposed. The non-transitory computer-readable data medium may be part of the puncture guiding unit and the puncture guiding unit may comprise a processor such as a central processing unit (CPU) for executing the executing the steps of the computer program described herein.

According to the present invention, also a use of the puncture guiding unit described herein, the puncture guiding system described herein or the puncture assistance system described herein for assisting a user in carrying out a puncture of a patient with a medical instrument is proposed.

### Definitions

A puncture is a targeted insertion of a medical instrument into the human body. This means that the medical instrument is inserted into the body of a patient and directed to a target location inside the patient's body. Having reached the target location it is possible that energy is applied, e.g. for ablating tissue, or that liquid or tissue samples are collected, or that medication is injected.

A medical instrument is in particular a cannulated medical instrument, for example, a hollow needle. Alternatively, the medical instrument can be a needle-shaped probe that, e.g., is configured for interstitial thermotherapy.

A puncture position is a position on a skin surface of a patient at which the medical instrument shall be inserted into a patient. The puncture position is defined by a coordinate in a coordinate system associated with a patient, e.g. a coordinate system of an imaging system. A planned puncture position is a position on a skin surface of a patient for inserting a medical instrument into a patient's body. The planned puncture position is determined manually or automatically, e.g., software-based, in a 2D or 3D image such as a tomographic image or the like showing the skin surface of a patient. The coordinate of the planned puncture position on the surface of the patient is defined in the coordinate system of the imaging system.

A puncture depth indicates the distance from the puncture position on a skin surface to the target location inside a patient's body. The puncture depth thus provides how deep the medical instrument is to be inserted into the patient to reach the target location when starting from the puncture position. Accordingly, a planned puncture depth indicates the planned distance from the planned puncture position to the planned target location inside the patient's body and can be determined manually or automatically in a 2D or 3D image of the patient. The planned puncture depth can be represented by an amount of a vector defined in the coordinate system of the imaging system.

A puncture orientation indicates the direction along which a medical instrument shall be inserted into a patient at the puncture position on the skin surface to reach a target location inside the patient's body. The puncture orientation preferably is defined relative to the skin surface of the patient. Accordingly, a planned puncture orientation indicates the planned direction along which the medical instrument shall be inserted into the patient at the planned puncture position to reach the planned target location inside the patient's body and is determined manually or automatically in a 2D or 3D image of the patient. The planned puncture orientation can be represented by a vector defined in the coordinate system of the imaging system.

The process of planning refers to a selection of at least one of a planned puncture position, a planned puncture depth and a planned puncture orientation in the coordinate system of the imaging system. The selection can be performed manually by a user by using an input device for selecting at least one of a planned puncture position, a planned puncture depth and a planned puncture orientation in a tomographic image visualized on a display. Alternatively, the selection can be performed automatically, e.g., software-based. Such a computer program configured for selecting at least one of a planned puncture position, a planned puncture depth and a planned puncture orientation may comprise an artificial neural network that is trained for receiving a tomographic image as input and for outputting at least one of a planned puncture position, a planned puncture depth and a planned puncture orientation for this tomographic image. The result of the planning process, i.e., the selection of at least one of a planned puncture position, a planned puncture depth and a planned puncture orientation in the coordinate system of the imaging system may be represented by planning data.

An imaging system configured for recording a tomographic image may be an X-ray device, e.g., a C-arm or a computed tomography (CT) scanner, a magnetic resonance imaging (MRI) scanner, a sonography device, or the like. A tomographic image recorded by such an imaging system may be represented by tomography image data. Herein, the term "tomography image data" is also used for fluoroscopic image data, which is not tomography image data in the narrower sense, but generated by an imaging system like a C-arm, for example. Accordingly, in the following, the term "tomographic image" means all images generated by an imaging system, i.e. also fluoroscopic images, for example from a C-arm. A tomographic image reconstructed from the tomography image data can be a two-dimensional or a three-dimensional tomographic image.

Once a tomographic image has been recorded from a patent with the imaging system, a position and orientation of the patient is known in the coordinate system of the imaging system. Movements of the patient relative to the imaging system after the recording of a tomographic image may give rise to uncertainty of the actual position and orientation in the coordinate system of the imaging system, e.g., at the time of carrying out a puncture. It may thus be advantageous to verify or correct the position and orientation of the patient relative to the imaging system when comparing the position and orientation of the patient of generating the tomographic image and at the later time of carrying out a puncture. Such a verification or correction may be possible based on tracking movements, e.g., visually, of a patient when generating the tomographic image and when carrying out a puncture.

In case, a patient moves relative to the imaging system after a tomographic image has been generated, patient registration may be required. Patient registration may be carried out using the optical target device or a camera, e.g., that is mounted to the imaging system. For example, a video of the patient that is recorded by the optical target device may be used for patient registration. The optical target device can thus be used for patient registration and for assisting a user in correctly aligning a medical instrument with the viewing axis.

Patient registration may be achieved by employing a patient registration marker that is detectable in both, a tomographic image and in a video. Such a patient registration marker, preferably, is arranged on the patient while generating the tomographic image and stays on the patient while recording a video of the patient. Preferably, the patient registration marker is arranged on the patient in such a way that it follows a movement of the patient, so that there is no relative movement between the patient and the patient registration marker. For example, the patient registration marker can be adhesively attached to the patient. For registration of the patient, a position of or deformation of the patient registration marker caused by movement of the patient can be detected when comparing the tomographic image to a later recorded video. Based on the detected deviation of the patient registration marker position or shape relative to the skin surface of the patient, the patient registration can be carried out. Patient registration may induce determining a transformation between a coordinate of the puncture position and/or puncture orientation at the time of generating the tomographic image and a coordinate of the puncture position and/or puncture orientation at the time of carrying out the puncture. Based on this transformation, puncture position, puncture orientation and/or puncture depth can be determined in the coordinate system of the imaging system at the time of carrying out the puncture. Preferably, the optical target device such as a camera is then positioned relative to the imaging system based on the determined transformation.

An optical target device is configured to provide an optical axis between a user and a patient to be punctured. An optical target device has a field of view with a viewing axis. A viewing axis, preferably, is the axis of symmetry of the field of view of the camera. However, it is also possible that the viewing axis is not coaxially aligned with respect to the optical target device. A viewing axis that is not coaxially aligned with respect to the optical target device may be realised with an optical target device having two reticles at least one of which is not located on the central axis of the optical target device, i.e., at least one of the two reticles is radially displaced with respect to the other reticle.

In particular, the optical target device may be a camera with a lens that has the optical axis or a tube with two reticles longitudinally displaced with respect to each other. In particular, an optical target device may comprise a tube with open ends such that a user can look through the tube to see the patient to be punctured. For adjusting a visual axis of such an optical target device, the tube may have two reticles that can be brought into superposition. Preferably, the optical target device is or is part of a portable electronic device such as smartglasses, a head-up display or a handheld device such as a digital camera, a smartphone or a tablet computer or the like. In particular, the optical target device may be or may comprise a camera. The camera has a field of view representing that part of the world that is visible through the camera. The camera also has a viewing axis that is the viewing direction of the camera perpendicular to the centre of a camera lens. The viewing axis of the camera thus runs along the centre of the camera's field of view. For example, in case the field of view has a circular cross-section, the viewing axis runs through the centre of the circular cross-section. A camera may generate video data representing a video of that part of the world that is visible through the camera. The video captured by the camera is defined in a coordinate system of the camera.

The optical target device such as a camera preferably is attached to or mounted on an optical target device holder. Preferably, the optical target device holder is attached to the imaging system. In case the optical target device holder is attached to the imaging system, the position of the junction at which the optical target device holder is attached to the imaging system is known in the coordinate system of the imaging system. A change position and orientation of the optical target device and/or an optical target device holder in space in the coordinate system of the imaging system can be determined, e.g., tracked. For example, a change position and orientation of the optical target device and/or an optical target device holder can be determined relative to the junction at which the optical target device holder is attached to the imaging system. The determined position of the optical target device and/or an optical target device holder may be represented by positioning data indicative of a position and/or orientation of the optical target device and/or an optical target device holder in the coordinate system of the imaging system.

It may be necessary to perform a registration process of the optical target device, e.g., in case, an optical target device is not attached via an optical target device holder to the imaging unit. An optical target device registration process includes determining a transformation between a coordinate in the coordinate system of the imaging system and a coordinate in the coordinate system of the optical target device. After registration, a position and orientation of the optical target device can be determined in the coordinate system of the imaging system. This allows tracking a position and orientation of the optical target device relative to the imaging system in the coordinate system of the imaging system. For example, a registration may be achieved by determining the position and orientation of the optical target device relative to the imaging system. This may be achieved by analysing that part of the world that can be seen through the optical target device. Furthermore, the optical target device may be brought to a position in space of which the coordinate is known in the coordinate system of the imaging system. Moreover, it is possible that the optical target device is equipped with a position sensor the position of which can be determined relative to a position the coordinate of which is known in the coordinate system of the imaging system, e.g., a position of a further position sensor associated with the imaging system.

The position and orientation of the optical target device in the coordinate system of the imaging system can be tracked, e.g., by evaluating that part of the world that can be seen through the optical target device, e.g., relative to the imaging system. Furthermore, motion tracking technologies based on optical sensors, accelerometers, GPS, gyroscopes, solid state compasses, radio-frequency identification (RFID), BLE (Bluetooth Low Energy) beacons can be employed in order to determine a position and/or orientation of the optical target device relative to the imaging system.

Positioning of the optical target device refers to the process of bringing the optical target device to a position and orientation in space relative to the imaging modality such that the puncture position on a patient's skin surface is within the field of view of the optical target device. The puncture position on the skin surface of the patient is known in the coordinate system of the imaging system once a tomographic image of the patient has been generated and a planned puncture position of the patient has been determined in the generated tomographic image. Positioning the optical target device relative to the imaging system such that the puncture position is within the field of view of the optical target device is possible since after registration, the position and orientation of the optical target device is known in the coordinate system of the imaging system. When looking at that part of the world that can be seen though the optical target device, a user thus sees the puncture position on the skin surface of the patient. For example, a user may see that part of the world that can be seen though the camera by looking through a viewfinder or the like of the camera or by looking at a video generated from video data recorded by the camera. Preferably, the optical target device is positioned relative to the patient such that the puncture position and puncture orientation for a medical instrument as planned via a tomographic image of a patient at least approximately coincide with a trajectory corresponding to the viewing axis of the optical target device. In this case, when a user looks at that part of the world that can be seen though the optical target device, in the centre of the field of view, the user directly looks at the puncture position and along the puncture orientation as planned o via the tomographic image of the patient.

Medical instrument positioning refers to the positioning of the medical instrument relative to a patient for performing a puncture at the puncture position as planned via a tomographic image of the patient. The medical instrument may be positioned manually or automatically, e.g., by a medical instrument positioning system, such that the medical instrument points with its distal end towards the puncture position on the skin surface of the patient and is oriented along the puncture orientation relative to the patient's skin surface. In case, the optical target device is positioned relative to the patient such that the puncture position and puncture orientation for a medical instrument as planned via a tomographic image of a patient lie on a trajectory corresponding to the viewing axis of the optical target device, the medical instrument can be positioned such that its orientation in space coincides with the viewing axis of the optical target device. When the medical instrument is positioned such that its orientation in space coincides with the viewing axis of the optical target device, when looking along the viewing axis, only a proximal end of the medical instrument is visible and not its distal end. Thereby, it is possible for a user to verify that a medical instrument is positioned correctly, i.e., with its distal end pointing towards the puncture position and with the medical instrument being oriented along the puncture orientation as planned.

It shall be understood that the aspects described above, and specifically the puncture guiding unit of claim 1, the puncture guiding system of claim 4, the puncture assistance system of claim 11, method of claim 13 and the computer program of claim 14, have similar and/or identical preferred embodiments, in particular as defined in the dependent claims.

It shall be understood that a preferred embodiment of the invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1a:: exemplary and schematically shows a puncture assistance system for assisting a user in carrying out a puncture of a patient's body;
- Fig. 1b:: exemplary and schematically shows the puncture guiding unit of the puncture assistance system in more detail;
- Fig. 2:: exemplary and schematically shows a puncture guiding system comprising an arch element holding a movable camera that can be used in conjunction with the puncture assistance system described with reference to Fig. 1a;
- Fig. 3a): exemplary and schematically shows a camera and its field of view with the viewing axis in a side view;
- Fig. 3b): exemplary and schematically shows a camera and its field of view with the viewing axis from behind the camera, e.g., from the viewing perspective of a user looking at the display of the camera;
- Fig. 4:: exemplary and schematically shows a puncture guiding system comprising an arch element holding multiple cameras at stationary positions that can be used in conjunction with the puncture assistance system described with reference to Fig. 1a;
- Fig. 5:: exemplary and schematically shows a puncture guiding system comprising a manually movable robot arm that holds a camera that can be used in conjunction with the puncture assistance system de-scribed with reference to Fig. 1a; and
- Fig. 6:: shows a flowchart diagram representing a method of assisting a user in carrying out a puncture of a patient's body.

### DETAILED DESCRIPTION OF EMBODIMENTS

### Puncture assistance system

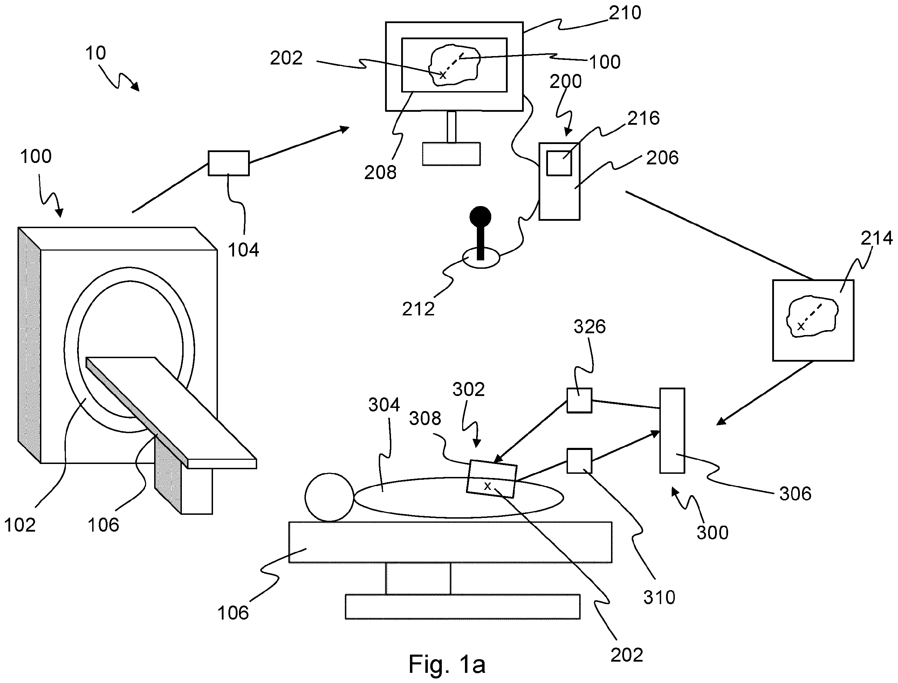

An exemplary puncture assistance system 10 is schematically shown in figure 1a.

The puncture assistance system 10 comprises an imaging system that is a computed tomography (CT) scanner 100 having a rotatable X-ray tube and a row of detectors placed in a gantry 102 to measure X-ray attenuations by different tissues inside a patient's body. Based on CT scans recorded by the CT scanner 100, tomography image data 104 representing a tomographic image of a scanned body part of a patient can be generated using tomographic reconstruction algorithms. The CT scanner 100 comprises a motorized CT table 106 on which a patient can be positioned. A patient lying on the CT table 106 can be moved into the gantry 102 such that a certain part of the patient is within the field of view of the X-ray tube. Of this body part arranged in the field of view of the X-ray tube, a tomographic image can be generated.

Alternatively, instead of the CT scanner 100, the puncture assistance system 10 may comprise, e.g., a magnetic resonance imaging (MRI) scanner for generating tomography image data representing a tomographic image of a scanned body part of a patient using tomographic reconstruction algorithms.

The puncture assistance system 10 further comprises a planning unit 200 that is configured to allow planning a puncture of a patient. The planning unit 200 may be configured such that planning a puncture of a patient can be performed manually by a user or automatically, e.g., without or substantially without intervention of a user.

In particular, the planning unit 200 may be configured to enable a selection of a planned puncture position 202 and/or a planned puncture orientation 204 and/or a planned puncture depth for carrying out a puncture of the patient's body. To this end, the planning unit 200 may be configured to receive the tomography image data 104 recorded for a scanned body part of the patient from the CT scanner 100 and to process the tomography image data 104 with a data processing device 206 such as a personal computer or the like. For example, the planning unit 200 may be configured to receive input from a user that selects a planned puncture position 202 and/or a planned puncture orientation 204 and/or a planned puncture depth into the patient's body in a tomographic image 208 visualized from the tomography image data 104. For example, a user may select with a cursor a planned puncture position 202 and/or a planned puncture orientation 204 and/or a planned puncture depth in the tomographic image 208. To this end, the tomographic image 208 may be displayed on a screen 210 and the user may select with a cursor a puncture position and/or a puncture orientation using an input device 212 such as a computer mouse or a joystick.

Based on a manual selection of a planned puncture position 202 and/or a planned puncture orientation 204 and/or a planned puncture depth in the tomographic image 208 by a user or automatically, e.g., solely software-based without or substantially without involving action of a user, the planning unit 200 may provide, e.g., output or store, planning data 214 representing the planned puncture position 202 and/or the planned puncture orientation 204 and/or the planned puncture depth relative to the patient's body in the coordinate system of the CT scanner 100. For example, the planning data 214 may represent the planned puncture position 202 and/or the planned puncture orientation 204 and/or the planned puncture depth relative to the patient's body part of which the tomography image data 104 have been recorded. Accordingly, the planning data may represent a tomographic image of the patient superimposed with the planned puncture position 202 and/or the planned puncture orientation 204 and/orthe planned puncture depth in the coordinate system of the CT scanner 100.

The planned puncture position 202 and/or the planned puncture orientation 204 and/or the planned puncture depth may be included as digital representations in form of a marker or icon in the planning data 214. For example, a marker indicating the planned puncture position 202 may be configured as a digital reticle and a digital dashed line may be used for indicating the planned puncture orientation 204 and/or the planned puncture depth, as it is exemplary depicted in tomographic image 208 depicted on the screen 210 of the planning unit 200 shown in figure 1a.

As mentioned before, in addition or as an alternative to such manual selection capabilities, the planning unit 200 may be configured to automatically determine a planned puncture position 202 and/or a planned puncture orientation 204 and/or a planned puncture depth relative to the patient's body based on the tomography image data 104. For example, the planning unit 200 may comprise a computer program 216 that is configured to automatically determine a planned puncture position 200 and/or a planned puncture orientation 204 and/or a planned puncture depth relative to the patient's body based on the tomography image data 104. To this end, the computer program may include an artificial neural network or other classification software that is trained to receive tomography image data 104 as input, to determine a planned puncture position 202 and/or a planned puncture orientation 204 and/or a planned puncture depth relative to the patient's body and to provide as an output planning data 214 configured as described for the manual selection process before.

Furthermore, the puncture assistance system 10 comprises a puncture guiding system 300 that comprises at least one camera 302. Alternatively or in addition to a camera, the puncture assistance system 10 may comprise another optical target device. The camera 302 may be configured as smartglasses, a head-up display or a handheld device such as a digital camera, a smartphone or a tablet computer or the like. The camera 302 is configured to generate and provide video data 310 representing a video 312 of that part of the world that is in the field of view of the camera 302. The camera 302 may comprise or may be connected to a display 308 for visualising the video 312, preferably, in real-time or at least with only a comparatively small delay. A comparatively small delay may be a delay that substantially cannot be resolved by a human's eye.

The puncture guiding system 300 further comprises an optical target device holder 303 that is attached to the CT scanner 100 and that holds the camera 302. The optical target device holder 303 may be static like an arch element or may comprise elements that are movable relative to the CT scanner 100, for example, several arms that are interconnected by joints. Since the is attached to the CT scanner 100, the position and orientation of the optical target device holder 303 and the camera 302 is known in the coordinate system of the CT scanner 100. Moreover, once a tomographic image of a patient has been generated by the CT scanner 100, the position and orientation of the patient is also known in the coordinate system of the CT scanner 100. Furthermore, the planned puncture position 202 and/or a planned puncture orientation 204 and/or a planned puncture depth are also defined in the coordinate system of the CT scanner 100.

Therefore, it is possible to select a position and orientation of the camera 302 in the coordinate system of the CT scanner 100, at which the puncture position and/or the puncture orientation and/or the puncture depth on the skin surface of the patient coincide with the viewing axis of the camera 302. For example, in case the optical target device holder 303 is movable, e.g., the optical target device holder 303 is configured as a robot arm, the camera 302 mounted to the optical target device holder 303 can be moved to the position in space where the puncture position and/or the puncture orientation and/or the puncture depth on the skin surface of the patient lie on the viewing axis of the camera 302.

In case the optical target device holder 303 is static but the camera 302 can be moved relative to the optical target device holder 303, e.g., along a track, the camera 302 can be moved along the optical target device holder 303 such that the puncture position and/or the puncture orientation and/or the puncture depth on the skin surface of the patient match with or are at least close to the viewing axis of the camera 302. In case there are several cameras 302 arranged on a static optical target device holder 303, that camera 302 of the several cameras 302 can be selected, for which the puncture position and/or the puncture orientation and/or the puncture depth on the skin surface of the patient match with or are at least close to the viewing axis of the camera 302. For controlling the positioning of the camera 302 relative to the CT scanner 100 such that the puncture position and/orthe puncture orientation and/or the puncture depth on the skin surface of the patient as planned lie on the viewing axis of the camera 302, the puncture guiding system 300 comprises a puncture guiding unit 306 that may be or may comprise a personal computer or another computing device.

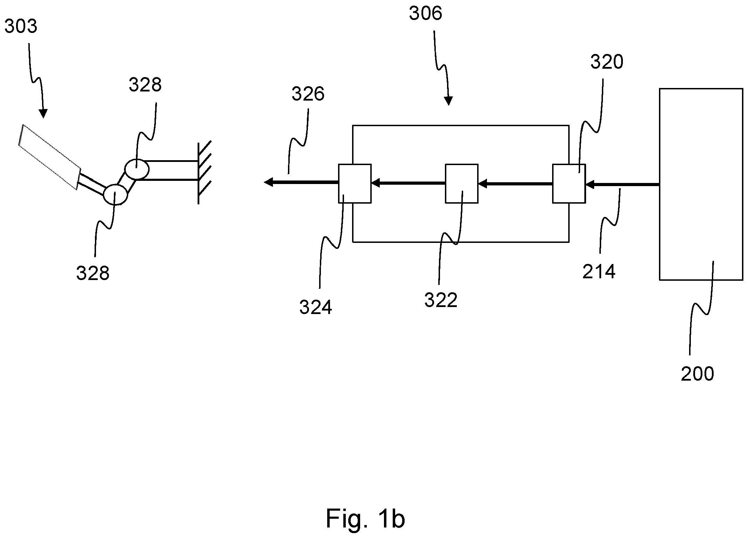

As depicted in more detail in figure 1b, the puncture guiding unit 306 has an input unit 320 that is configured for receiving planning data 214 from the planning unit 200. The planning data 214 are indicative of the puncture position 202 and/or the puncture orientation 204 in a coordinate system of the CT scanner 100. The puncture guiding unit 306 comprises a position determination unit 322 that is configured for determining for the camera 302 a target position and/or orientation in the coordinate system of the CT scanner 100 at which the viewing axis 414 points towards the puncture position 202 and along the puncture orientation 204 relative to the skin surface of the patient 304 using the planning data 214. Moreover, the puncture guiding unit 306 comprises an output unit 324 that is configured for providing guidance and/or selection data 326 indicative of the determined target position and/or orientation of the camera 302 relative to the patient 304. The guidance and/or selection data 326 can be used for controlling the optical target device holder 303. For example, in case the optical target device holder 303 is a robot arm comprising joints 328 having brakes and or actuators, the guidance and/or selection data 326 can be used for controlling a movement of the joints 328 or for guiding a user when moving the robot arm 303.

Preferably, for controlling the positioning of the camera, the puncture guiding unit 306 is configured for processing the planning data 214 and optionally, also video data 310 and/or positioning data representing a position and/or orientation of the camera 302 in the coordinate system of the CT scanner 100 and for providing the guidance and/or selection data 326. For generating positioning data, the camera 302 and/or the optical target device holder 303 may be tracked using motion tracking technologies based on, e.g., optical sensors, accelerometers, GPS, gyroscopes, solid state compasses, radio-frequency identification (RFID). Thereby, it is possible with the puncture guiding system 300 to indicate the puncture position and/or the puncture orientation and/or puncture depth on the patient's skin surface , e.g., for a user that carries out the puncture. For carrying out the puncture, a user may then arrange the medical instrument such that it is aligned with the viewing axis of the camera. In case the medical instrument is aligned with the viewing axis of the camera, the user knows that the medical instrument is arranged relative to the patient and can be inserted at the puncture position along the puncture orientation into the patient as planned previously in the tomographic image of the patient.

### a) Puncture guiding system comprising an arch element holding a movable camera

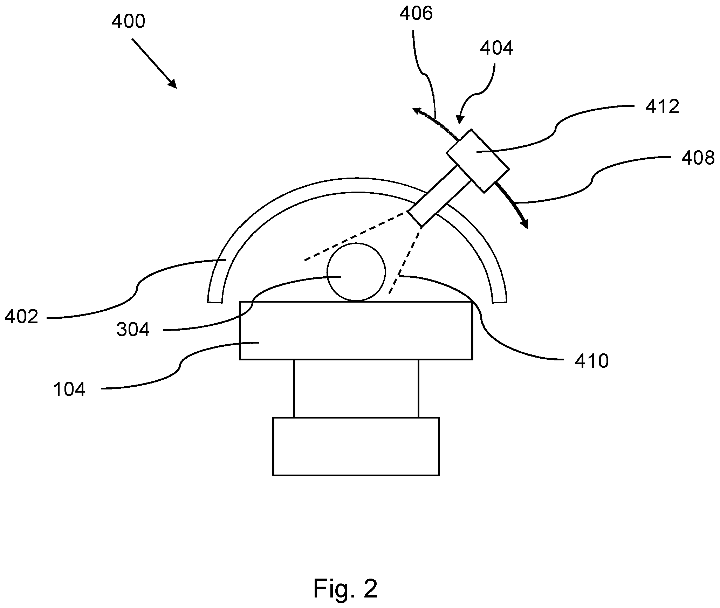

Figure 2 schematically shows an exemplary embodiment of a puncture guiding system 400 that can be used as the puncture guiding system 300 described with references to figure 1a.

The puncture guiding system 400 comprises an optical target device holder that is configured as an arch element 402 that may have the shape of a semi-circle and that is arranged to span over the CT table 104 of the CT scanner 100. In operation, thereby, a patient 304 lying on the CT table 304 is positioned to be at least partly surrounded by the arch element 402.

The arch element 402 holds a camera 404 that may be the camera 302 described with reference to figure 1a. Alternatively or in addition to a camera, another optical target device may be employed. The camera 404 can be held movably at the arch element 402 such that the camera 404 can be moved along the arch element 402 as indicated by the arrows 406, 408, e.g., along a track. Thereby, a field of view 410 of the camera 404 can be changed relative to the patient 304. By moving the camera 404 along the arch element 402 it is thus possible capture a different body part of the patient 304. It may also be possible that the puncture guiding system 400 can be moved along the long side of the CT table 104 such that further body parts of the patient 304 can be captured with the puncture guiding system 400. The same effect can be achieved if the CT table 104 is moved relative to the puncture guiding system 400 such that a different body part of the patient 304 is moved into the field of view 410 of the camera 404. Since the arch element 402 is attached to the CT scanner 100, its position and also the position of the camera 404 held by the arch element 402 is known in the coordinate system of the CT scanner 100.

The camera 404 has a display 412 that can directly visualize a video generated from the video data recorded by the camera 404. Actuators of the puncture guiding system 400 may be controlled based on guidance and/or selection data 326 to move the camera 404 along the arch element 402 to a position where the puncture position and/or puncture orientation into the patient 304 is within the field of view 410 of the camera 404. If necessary, the CT table 104 and the puncture guiding system 400 may be moved relative to each other in addition in order to move the camera 404 to a position where the puncture position and/or puncture orientation into the patient 304 is within the field of view 410 of the camera 404. Having reached the final camera position, a confirmation signal may be provided by the puncture guiding system 400 that can be, e.g., a visual, haptic and/or an acoustic signal.

Alternatively to controlling actuators, the camera 404 may also be held on the arch element 402 such it can be moved manually by a user along the arch element 402, e.g., along a track, and can be fixed at a selected position. In order to find the position where the puncture position and/or puncture orientation into the patient 304 is within the field of view 410 of the camera 404, feedback may be provided to a user, e.g., based on guidance and/or selection data 326. For example, visual and/or acoustic feedback may be provided by the puncture guiding system 400 hat is configured to guide the user to position the camera such that the puncture position and/or puncture orientation is within the field of view 410 of the camera 404. Moreover, haptic feedback may be provided by the puncture guiding system 400 in order to guide the user to correctly position the camera 404 relative to the patient 304. To provide haptic feedback, the arch element 402 may comprise brakes that can be actuated, e.g., based on guidance and/or selection data 326, to impede further movement of the camera 404 along the arch element 402. Thereby, the user can experience that the camera 404 should not be moved further but should be moved in the opposite direction to reach the position where the selected puncture position and/or puncture orientation into the patient 304 is within the field of view 410 of the camera 404.

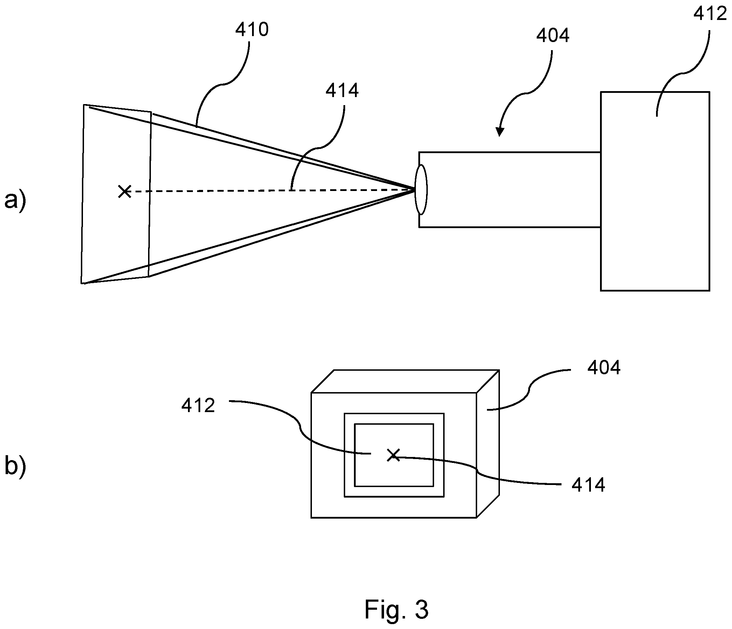

In operation, it is particularly preferred that the camera 404 is moved to a position relative to the patient 304 where the viewing axis 414 at least approximately points towards the puncture position and along puncture orientation i. This preferred positioning of the camera 404 as one example of an optical target device is visualized in more detail in figures 3a) and 3b). Figure 3a) shows the camera 404 and its field of view 410 with the viewing axis 414 in a side view. Figure 3b) shows the camera 404 and its field of view 410 with the viewing axis 414 from behind the camera, e.g., from the viewing perspective of a user looking at the display 412.

Having the viewing axis 414 superimposed with the puncture position and/or puncture orientation has the advantage that a medical instrument like a puncture needle can be oriented along the viewing axis 414 such that it can be more reliably introduced into the patient 304 at the puncture position and along the puncture orientation. This is because when aligning the medical instrument with the viewing axis 414, when looking along the viewing axis 414 only the proximal end of the medical instrument is visible whereas a shaft and a distal end of the medical instrument are not visible. Thus, when only seeing the medical instrument's proximal end, e.g., in a video on a display 412, a user can be relatively certain that the medical instrument will be introduced into the patient 304 at the puncture position and along the puncture orientation. In case a user still sees parts of the shaft or the distal end of the medical instrument, the user may recognize that the position of the medical instrument relative to the patient 304 needs to be corrected in order to have the medical instrument aligned with the viewing axis 414. The puncture guiding system 400 may comprise a laser that is arranged and configured to provide a laser beam along the viewing axis 414. Thereby, the puncture guiding system 400 may be configured to visually indicate the viewing axis 414 to a user. In case, a user can see the laser beam impinging only on the proximal end of the medical instrument, the user can be certain that the medical instrument is arranged along the viewing axis 414. When additionally providing a laser beam indicating the viewing axis 414, a puncture may be carried out with improved reliability and/or accuracy. Alternatively or additionally to making use of a laser, the start or the trajectory of the viewing axis 414 may be indicated directly on the display. For example, reticle or a physical marker, e.g., a sticker, may be applied onto the display. It is also possible to include a digital representation of a marker in the video that indicates the start of the viewing axis 414. In both cases, when a user brings the medical instrument in superposition with the physical or digital marker such that the full medical instrument is arranged along a virtual line (the viewing axis 414) starting from the marker, a user can be relatively certain that the medical instrument is arranged along the viewing axis 414 and thus correctly positioned relative to the patient for performing the puncture as planned.

### b) Puncture guiding system comprising an arch element holding multiple cameras at stationary positions

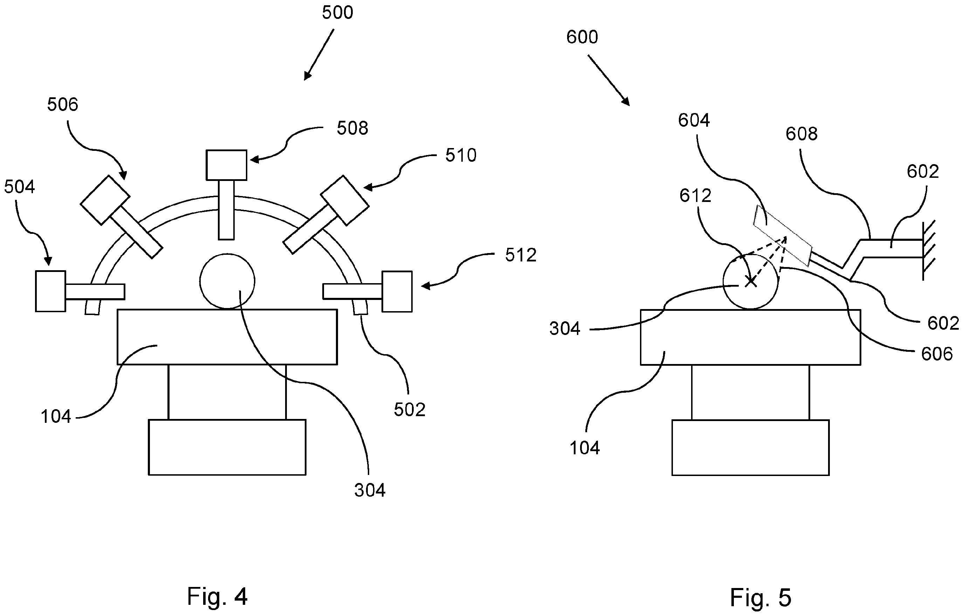

Figure 4 schematically shows another exemplary embodiment of a puncture guiding system 500 that can be used as the puncture guiding system 300 described with references to figure 1a.

The puncture guiding system 500 comprises an optical target device holder that is configured as an arch element 502 that may have the shape of a semi-circle and that is arranged to span over the CT table 104 of the CT scanner 100. In operation, thereby, a patient 304 lying on the CT table 304 is positioned to be at least partly surrounded by the arch element 502. The arch element 502 is equipped with multiple cameras 504, 506, 508, 510, 512 that are arranged along the length of the arch element 502. Alternatively or in addition to multiple cameras, other optical target devices may be used in conjunction with the arch element 502. The multiple cameras 504, 506, 508, 510, 512 are arranged at fixed positions along the arch element 502, i.e., the multiple cameras 504, 506, 508, 510, 512 are stationary. Thereby, each of the multiple cameras 504, 506, 508, 510, 512 can capture a video of a different body part of the patient 304, i.e., from a different viewing perspective. Since the arch element 502 is attached to the CT scanner 100, its position and also the position of the multiple cameras 504, 506, 508, 510, 512 held by the arch element 502 are known in the coordinate system of the CT scanner 100.

Based on planning data received from a planning unit, a specific camera of the multiple cameras 504, 506, 508, 510, 512 may be selected by the puncture guiding system 500 for which the puncture position and/or puncture orientation into the patient 304 are located within the camera's field of view 410. In case the puncture position and/or puncture orientation into the patient 304 are located within the field of view of two or more cameras, preferably, the puncture guiding system 500, e.g., its puncture guiding unit, preferably, is configured for selecting that camera of the of two or more cameras for which the puncture position and/or puncture orientation lies closest to its viewing axis, cf. figures 3a) and 3b).

The selection of that camera for which the viewing axis at least approximately coincides with the determined puncture position and the puncture orientation may be indicated to a user, e.g., visually and/or acoustically by the puncture guiding system 500, e.g., based on guidance and/or selection data 326. A user may than use the display of that selected camera to carry out the puncture of the patient 304. By using that camera for which the puncture position and/or puncture orientation coincide with the viewing axis, the user can arrange the medical instrument such that when looking along the viewing axis, only the proximal end of the medical instrument is visible. As mentioned before, the viewing axis of the camera to be used may be indicated to a user using a respectively arranged laser of the puncture guiding system 500. Additionally or alternatively, a reticle, a physical or digital marker on the display may be used for indicating the start of the viewing axis. Thereby, the user can be relatively sure that the medical instrument is introduced into the patient's body at the selected puncture position and along the selected puncture orientation.

It may be that the more cameras are available and the closer these cameras are arranged with respect to each other along the length of the arch element 502, the more likely is a situation in which the selected puncture position and/or puncture orientation are located actually on a viewing axis of a field of view of at least one of these cameras. For example, a plurality of cameras 504, 506, 508, 510, 512 may be arranged on the arch element 502 so that at least every 10° or even less along the arch element 502, a camera of the multiple cameras 504, 506, 508, 510, 512 is arranged.

### c) Puncture guiding system comprising a manually movable robot arm that holds a camera

Figure 5 schematically shows another exemplary embodiment of a puncture guiding system 600 that can be used as the puncture guiding system 300 of the puncture assistance system 10 described with references to figure 1a.

The puncture guiding system 600 comprises an optical target device holder that is configured as a robot arm 602 that holds a camera 604, e.g., a tablet computer or a smartphone equipped with a camera. Alternatively or in addition to camera 604, another optical target device may be mounted to the robot arm The robot arm 602 has several elements connected by joints 606, 608 so that it can be moved manually relative to the CT table 104 and thus also relative to a patient 304 lying on the CT table 104. With the robot arm 602, it is thus possible to relatively freely position the camera 604 in space relative to the patient 304. The robot arm 602 is attached to the CT scanner 100 such that the position of the robot arm 602 and the camera 604 held by the robot arm 602 is known in the coordinate system of the CT scanner 100.

As described before, for carrying out a puncture, it is particularly preferred that the camera 604 is positioned such that the puncture position and/or puncture orientation into the patient 304, e.g., as determined with a planning unit, is located within the field of view 610 of the camera 604, e.g., based on guidance and/or selection data 326. Even more preferably, for carrying out a puncture, the camera 604 is positioned such that the viewing axis 612 at least approximately points towards the puncture position and along puncture orientation into the patient 304 , e.g., based on guidance and/or selection data 326. This is because if the selected puncture position and/or puncture orientation into the patient 304 at least approximately coincides with the viewing axis 612, a user can comparatively easily find the puncture position and/or puncture orientation into the patient 304 by placing the medical instrument in the field of view of the camera 604 such that, only the proximal end of the medical instrument is visible and not the shaft and the medical instrument's distal end. Accordingly, it is also relatively easy for a user to recognize an incorrect positioning of the medical instrument and to correct the position of the medical instrument such that it can be correctly inserted into the puncture position and along the puncture orientation. As mentioned before, the positioning of the medical instrument along the viewing axis of the camera may be supported by a laser that is arranged and configured for visually indicating the viewing axis to a user. Additionally or alternatively to a laser, a reticle, or physical or digital markers may be applied, e.g., on the display, to indicate the start of the camera's viewing axis.

With the robot arm 602, a manual positioning of the camera 604 held by the robot arm 602 can be achieved in different ways.

For example, a puncture guiding unit may receive planning data from a planning unit. Based on the planning data, the planning unit may assist in the positioning of the camera 604 relative to the patient so that the puncture position and/or puncture orientation into the patient 304 is located within the camera's field of view 610 and even more preferably coincide with the viewing axis 612. To this end, the position of the camera can be tracked, i.e., the position information of the camera can be continuously updated.

For assisting a user in finding the correct position of the camera 604, the puncture guiding system 600 may provide guidance by generating an acoustic and/or visual signal that is indicative of where to move the camera 604 in space in order to reach the intended camera position, e.g., based on guidance and/or selection data 326. In case the correct position is reached with the camera 604 upon manually moving the robot arm 602, the puncture guiding system 600 may be configured for providing a confirmation signal that can be an acoustic, haptic and/or a visual signal. Once the correct position is reached, a user can safely carry out the puncture as planned.

Alternatively or in addition to optical and/or acoustic guidance, the puncture guiding system 600 may be configured to provide haptic guidance to a user, e.g., based on guidance and/or selection data 326. Haptic guidance may be accomplished by providing haptic feedback to a user while manually moving the robot arm 602 in space in orderto find the correct position of the camera 604 for carrying out the puncture. A possible implementation of haptic feedback makes use of brakes and/or actuators that are arranged at the robot arm 602 in order to impede or enable manual movement of the robot arm 602 in certain directions in space. Accordingly, in case the robot arm 602 is moved manually away from the intended camera position, the brakes and/or actuators of the robot arm 602 may be controlled by a puncture guiding unit in order to impede a further movement away from the planned camera position. On the contrary, in case the robot arm 602 is moved manually towards the intended camera position, the brakes and/or actuators of the robot arm 602 may be controlled in order to enable a further movement towards the planned camera position. In case a user has reached the intended camera position for carrying out the puncture, a visual, acoustic and/or haptic confirmation signal may be provided.

### d) Puncture guiding system comprising an automatically movable robot arm that holds a camera

The puncture guiding system 600 described with reference to figure 5 can also be modified in a way that the robot arm 602 can move automatically, i.e., by employing one or more motors, e.g., based on guidance and/or selection data 326.

The puncture guiding unit may be configured for generating a control signal, e.g., based on guidance and/or selection data 326, that is suitable for controlling the one or more motors in order to move the camera 604 with the robot arm such that the puncture position and/or puncture orientation into the patient 304 is located within the camera's field of view 610 or even coincide with the viewing axis 612.

In case the puncture guiding system 600 with manually or automatically movable robot arm 602 is to be used with the puncture assistance system 10 comprising an MRI scanner instead of the CT scanner 100, the robot arm 602 is preferably made of a MRI-compatible material such as plastics.

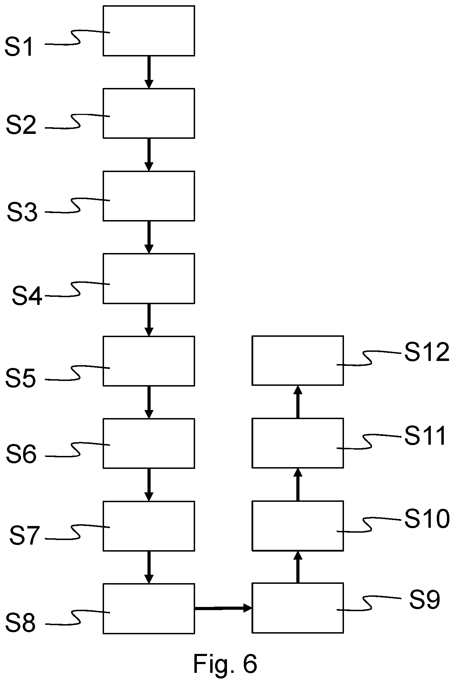

Figure 6 shows a flowchart diagram representing a method of assisting a user in carrying out a puncture of a patient's body. The method can be carried out, e.g., using the puncture assistance system as described with reference to figures 1a to 5.

In the method, an imaging system such as a CT scanner or a MRI scanner is provided (step S1) and a patient is positioned onto a table of the imaging system, e.g., a CT table or an MRI table (step S2). The patient is then moved with the CT table into the field of view of the imaging system (step S3). With the imaging system, tomography image data of a body part of the patient that is in the field of view of the imaging system are generated (step S4). From the generated tomography image data, a tomographic image of the patient's body part is generated using a tomographic reconstruction algorithm (step S5).

Based on the tomographic image, a puncture position and/or puncture orientation and/or puncture depth is planned (step S6). The planning of the puncture position and/or puncture orientation and/or puncture depth can be accomplished in various ways, for example, manually by a user that uses an input device for selecting with a cursor a puncture position and/or a puncture orientation in the tomographic image. The selected puncture position and/or a puncture orientation is then stored together with the tomographic image as planning data (step S7). As an alternative approach, puncture position and/or a puncture orientation and/or puncture depth in the tomographic image may be selected automatically without or substantially without intervention of a user by a computer program. In either way, a puncture position and/or a puncture orientation and/or puncture depth is preferably determined in such a way that the distance to be covered by the medical instrument inside the patient in order to reach a target location is as short as possible. A puncture position and/or a puncture orientation and/or puncture depth is preferably determined in such a way that sensitive tissue is not damaged during a puncture. A computer program used for determining a puncture position and/or a puncture orientation and/or puncture depth in the tomographic image may comprise an artificial neural network that is trained for finding a puncture position and/or a puncture orientation and/or puncture depth that causes relatively low harm to a patient and also provides a relatively low risk of damaging sensitive tissue of the patient. As a further alternative approach, puncture position and/or a puncture orientation and/or puncture depth in the tomographic image may be selected both, automatically and manually. For example, a computer program may first select a puncture position and/or a puncture orientation and/or puncture depth in the tomographic image automatically. The selected puncture position and/or a puncture orientation and/or puncture depth may then be modified by a user manually and the resulting puncture position and/or puncture orientation is then stored together with the tomographic image as planning data.

Having recorded the tomography image data of the patient's body part, the position and orientation of the patient is known in the coordinate system of the imaging system.

Subsequently, the patient positioned on the table of the imaging system is moved relative to the imaging system such that the patient's body part of which the tomography image data have been recorded can be accessed by a user (step S8). Since the movement of the table is defined in the coordinate system of the imaging system, also after having moved the patient together with the table, the position and orientation of the patient is known in the coordinate system of the imaging system.

An optical target device such as a camera belonging to a puncture guiding system of the puncture assistance system is then moved such that the viewing axis of the optical target device at least approximately points towards the puncture position and along the puncture orientation as planned (step S9). This arrangment of the optical target device can be accomplished in various ways.

For example, the optical target device can be mounted to an optical target device holder that is attached to the imaging system. Since the optical target device holder is attached to the imaging system, also the positon and orientation of the optical target device is known in the coordinate system of the imaging system. The optical target device can then be moved relative to the optical target device holder or together with the optical target device holder such that puncture position and/or the puncture orientation as planned lie on or at least close to the viewing axis of the optical target device (step S10). Optionally, it is possible that the position of the optical target device holder and/or the optical target device is tracked relative to the imaging system, i.e., in the coordinate system of the imaging system.

Optionally, a patient registration marker that exhibiting the property that it can be recorded both fluoroscopically and optically can be used. To this end, the patient registration marker that is both fluoroscopically and optically detectable is positioned on the patient prior to generating the tomography image data of the patient's body part. The patient registration marker is then recorded together with the patient's body part such that the tomography image data represent the patient's body part together with the patient registration marker. The patient registration marker, preferably, is fixed to the patient such that it stays in positon when the patient's body part is brought into the field of view of the optical target device. Since the patient registration marker is also optically detectable, the patient registration marker can also be captured by the optical target device. When processing the video data of a camera, a relative movement of the patient relative to the imaging system can be detected based on the position of the patient registration marker in the tomographic image and the position of the patient registration marker in the video captured by the camera. Based on a detected deviation in the position of the patient registration marker caused by a movement of the patient, the puncture position and/or the puncture orientation may be corrected and the optical target device may be moved to a position at which the corrected puncture position and/or the puncture orientation coincide with the trajectory of the viewing axis of the optical target device.

To bring the optical target device to a target position and/or orientation relative to the patient, where the puncture position and/or a puncture orientation on the patient lie on or at least close to the viewing axis of the optical target device, the optical target device may be positioned on an arch element and can be moved along the arch element, e.g., manually or automatically by one or more actuators, such that the puncture position and/or a puncture orientation lie on the viewing axis of the optical target device. Alternatively, a plurality of optical target devices may be arranged along an arch element in a stationary manner and an optical target device may be selected from the plurality of cameras for which the puncture position and/or a puncture orientation lie the closest to the viewing axis of the optical target device. Yet, alternatively, the optical target device may be mounted on a robot arm that may be operated manually, i.e., by a user, or automatically, by means of actuators.

Having reached the target position and orientation of the optical target device such that the puncture position and/or the puncture orientation on the patient lie on or at least close to the viewing axis of the optical target device, a confirmation signal is provided that can be, e.g., a visual, haptic and/or an acoustic signal (step S11). A display of the optical target device, e.g., of a camera, then visualizes the patient's body part to be punctured such that the puncture position and/or the puncture orientation on the patient lie on or at least close to the viewing axis of the optical target device (step S12). The viewing axis may optionally be further visualized using a laser that emits a laser beam that substantially coincides with the viewing axis of the optical target device. Additionally or alternatively, a reticle, a physical or digital marker may be used with the display to indicate the optical target device's viewing axis.

The optical target device being positioned such that the viewing axis at least approximately points towards the puncture position and along the puncture orientation has the advantage that a user can positon a medical instrument such that only the medical instrument's proximal end is visible when looking along the viewing axis. Thereby, a user can be certain that the medical instrument is actually positioned such that it points towards the intended puncture position on the patient and along the puncture orientation. In case a user can see further parts of the medical instrument on the display, the user can corrected to position of the medical instrument such that only its proximal end is visible. This allows a comparatively easy and accurate puncturing of a patient.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or device may fulfil the functions of several items recited in the claims. The mere fact that certain measures are recited in mutually different dependent claims does not indicate that a combination of these measures cannot be used to advantage.

Procedures like determining the puncture position and/or the puncture orientation in a coordinate system of an imaging system, determining a position and/orientation of an optical target device etc. performed by one or several units or devices can be performed by any other number of units or devices. These procedures can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any units described herein may be processing units that are part of a classical compu-ting system. Processing units may include a general-purpose processor and may also include a field programmable gate array (FPGA), an application specific integrated circuit (ASIC), or any other specialized circuit. Any memory may be a physical system memory, which may be volatile, non-volatile, or some combination of the two. The term "memory" may include any computer-readable storage media such as a non-volatile mass storage. If the computing system is distributed, the processing and/or memory capability may be distributed as well. The computing system may include multiple structures as "executable components". The term "executable component" is a structure well understood in the field of computing as being a structure that can be software, hardware, or a combination thereof. For instance, when implemented in software, one of ordinary skill in the art would understand that the structure of an executable component may include software objects, routines, methods, and so forth, that may be executed on the computing sys-tem. This may include both an executable component in the heap of a computing sys-tem, or on computer-readable storage media. The structure of the executable component may exist on a computer-readable medium such that, when interpreted by one or more processors of a computing system, e.g., by a processor thread, the computing system is caused to perform a function. Such structure may be computer readable directly by the processors, for instance, as is the case if the executable component were binary, or it may be structured to be interpretable and/or compiled, for instance, whether in a single stage or in multiple stages, so as to generate such binary that is directly interpretable by the processors. In other instances, structures may be hard coded or hard wired logic gates, that are implemented exclusively or near-exclusively in hardware, such as within a field programmable gate array (FPGA), an application specific integrated circuit (ASIC), or any other specialized circuit. Accordingly, the term "executable component" is a term for a structure that is well understood by those of ordinary skill in the art of computing, whether implemented in software, hardware, or a combination. Any embodiments herein are described with reference to acts that are performed by one or more processing units of the computing system. If such acts are implemented in software, one or more processors direct the operation of the computing system in response to having executed computer-executable instructions that constitute an executable component. Computing system may also contain communication channels that allow the computing system to communicate with other computing systems over, for example, network. A "network" is defined as one or more data links that enable the transport of electronic data between computing systems and/or modules and/or other electronic devices. When information is transferred or provided over a network or another communications connection, for ex-ample, either hardwired, wireless, or a combination of hardwired or wireless, to a computing system, the computing system properly views the connection as a transmission medium. Transmission media can include a network and/or data links which can be used to carry desired program code means in the form of computer-executable instructions or data structures and which can be accessed by a general-purpose or special-purpose computing system or combinations. While not all computing systems require a user interface, in some embodiments, the computing system includes a user interface system for use in interfacing with a user. User interfaces act as input or output mechanism to users for instance via displays.

Those skilled in the art will appreciate that at least parts of the invention may be practiced in network computing environments with many types of computing system configurations, including, personal computers, desktop computers, laptop computers, message processors, hand-held devices, multi-processor systems, microprocessor-based or programmable consumer electronics, network PCs, minicomputers, main-frame computers, mobile telephones, PDAs, pagers, routers, switches, data centres, wearables, such as glasses, and the like. The invention may also be practiced in distributed system environments where local and remote computing system, which are linked, for example, either by hardwired data links, wireless data links, or by a combination of hardwired and wireless data links, through a network, both perform tasks. In a distributed system environment, program modules may be located in both local and remote memory storage devices.

Those skilled in the art will also appreciate that at least parts of the invention may be practiced in a cloud computing environment. Cloud computing environments may be distributed, although this is not required. When distributed, cloud computing environments may be distributed internationally within an organization and/or have components possessed across multiple organizations. In this description and the following claims, "cloud computing" is defined as a model for enabling on-demand network access to a shared pool of configurable computing resources, e.g., networks, servers, storage, applications, and services. The definition of "cloud computing" is not limited to any of the other numerous advantages that can be obtained from such a model when deployed. The computing systems of the figures include various components or functional blocks that may implement the various embodiments disclosed herein as explained. The various components or functional blocks may be implemented on a local computing system or may be implemented on a distributed computing system that includes elements resident in the cloud or that implement aspects of cloud computing. The various components or functional blocks may be implemented as software, hardware, or a combination of software and hardware. The computing systems shown in the figures may include more or less than the components illustrated in the figures and some of the components may be combined as circumstances warrant.

Any reference signs in the claims should not be construed as limiting the scope.

## Claims

1. A puncture guiding unit for a puncture guiding system for assisting a user in carrying out a puncture of a patient with a medical instrument at a puncture position and along a puncture orientation, the puncture guiding unit comprising
- an input unit that is configured for receiving planning data indicative of the puncture position and/or the puncture orientation in a coordinate system of an imaging system,
- a position determination unit that is configured for determining for an optical target device having a field of view with a viewing axis,
- a target position and/or orientation in the coordinate system of the imaging system at which the viewing axis at least approximately runs towards the puncture position and along the puncture orientation relative to the skin surface of the patient using the planning data, and
- an output unit that is configured for providing guidance and/or selection data indicative of the determined target position and/or orientation of the optical target device relative to the patient.

2. The puncture guiding unit according to claim 1, wherein the guidance and/or selection data are suitable for controlling one or more actuators and/or brakes that are arranged and configured for guiding the optical target device to the determined target position and/or orientation of the optical target device relative to the patient.

3. The puncture guiding unit according to claim 1 or 2, wherein the guidance and/or selection data are suitable for indicating a direction along which the optical target device has to be moved in order to bring the optical target device to the determined target position and/or orientation relative to the patient, or wherein the guidance and/or selection data are suitable for indicating that optical target device for which the determined target position and/or orientation of the optical target device relative to the patient is the closest to the viewing axis.

4. A puncture guiding system for assisting a user in carrying out a puncture with a medical instrument at a puncture position and along a puncture orientation on the patient's skin surface of a patient, the puncture guiding system comprising:
- an optical target device having a field of view associated therewith and a viewing axis,
- an optical target device holder that is configured for holding the optical target device relative to the patient, and
- the puncture guiding unit according to at least one of the preceding claims.

5. The puncture guiding system according to claim 4, wherein the optical target device is a portable electronic device comprising a camera.

6. The puncture guiding system according to claim 4 or 5, wherein the optical target device holder is configured as a robot arm with one or more joints.

7. The puncture guiding system according to claim 6, wherein the robot arm comprises one or more brakes that are arranged and configured to be controlled by the puncture guiding unit to impede further movement of the joints.

8. The puncture guiding system according to claim 6 or 7, wherein the robot arm comprises one or more actuators that are arranged and configured to be controlled by the puncture guiding unit for actuating the joints to enable a further movement of the joints.

9. The puncture guiding system according to claim 4 or 5, wherein optical target device holder is configured as an arch element that is configured to movably hold the optical target device such that the optical target device can be moved along the arch element.

10. The puncture guiding system according to claim 4 or 5, wherein optical target device holder is configured as an arch element that is configured to hold one or more optical target devices at a stationary position relative to the arch element.

11. A puncture assistance system for assisting a user in carrying out a puncture with a medical instrument at a puncture position and along a puncture orientation on the patient's skin surface of a patient, the puncture assistance system comprising:
- an imaging system that is configured for generating a tomographic image of a patient,
- a planning unit that is configured for generating planning data indicative of the puncture position and/or the orientation in a coordinate system of the imaging system based on the tomographic image, and
- the puncture guiding system according to at least of claims 4 to 10,
wherein the puncture guiding system preferably is attached to the imaging system.

12. A method for assisting a user in carrying out a puncture with a medical instrument at a puncture position and along a puncture orientation on the patient's skin surface of a patient, the method comprising the steps of:
- determining the puncture position and/or the puncture orientation in a coordinate system of an imaging system,
- determining a target position and/or orientation of an optical target device at which a viewing axis of the optical target device at least approximately runs towards the determined puncture position and along the puncture orientation relative to the skin surface of the patient, and
- guiding the optical target device to the determined puncture position and/or the puncture orientation such that the viewing axis of the optical target device at least approximately runs towards the determined puncture position and along the puncture orientation relative to the skin surface of the patient, or
- selecting that optical target device for which the viewing axis of the optical target device at least approximately runs towards the determined puncture position and/or the puncture orientation relative to the skin surface of the patient.

13. A computer program for assisting a user in carrying out a puncture with a medical instrument at a puncture position and along a puncture orientation of the patient's skin surface, the computer program being configured for executing the steps of:
- receiving planning data indicative of the puncture position and/or the orientation in a coordinate system of an imaging system,
- determining a target position and/or orientation of the optical target device in the coordinate system of the imaging system at which a viewing axis of the optical target at least approximately runs towards the puncture position and along the puncture orientation relative to the skin surface of the patient using the planning data, and
- providing guidance and/or selection data indicative of the determined target position and/or orientation of the optical target device relative to the patient,
when run on a computer.

14. A non-transitory computer-readable data medium storing the computer program of claim 13.

15. A use of the puncture guiding unit according to at least one of claims 1 to 3, the puncture guiding system according to at least one of claims 4 to 10 or the puncture assistance system according to claim 11 for assisting a user in carrying out a puncture of a patient with a medical instrument.
